# EUROPEAN PATENT SPECIFICATION

(11) **EP 4 032 082 B1**
(45) Date of publication and mention of the grant of the patent: **12.02.2025**
(21) Application number: 20864434.4
(22) Date of filing: 18.09.2020
(51) Int. Cl.: G09B 23/30, G09B 23/00, G09B 23/28, G09B 23/32, G09B 23/34

(54) **SYNTHETIC EYE MODEL FOR OCULAR IMPLANT SURGICAL TRAINING**
SYNTHETISCHES AUGENMODELL FÜR CHIRURGISCHES TRAINING EINES AUGENIMPLANTATS
MODÈLE D'OEIL SYNTHÉTIQUE POUR FORMATION CHIRURGICALE D'IMPLANT OCULAIRE

(30) Priority: 18.09.2019 US 201962902274 P
(43) Date of publication of application: 27.07.2022
(73) Proprietor: Alcon Inc., 1701 Fribourg (CH)
(72) Inventor: SCHIEBER, Andrew T., Irvine, CA 92618 (US); TRUONG, Hector, Irvine, CA 92618 (US); SUSSMAN, Shane, Irvine, CA 92618 (US); DUNPHY, Liam, Irvine, CA 92618 (US)
(74) Representative: Murgitroyd & Company
(86) International application number: PCT/US2020/051498
(87) International publication number: WO 2021/055751

(56) References cited:
- WO-A1-2019/106803
- US-A1- 2004 050 392
- US-A1- 2009 291 423
- US-A1- 2011 098 809
- US-A1- 2012 021 397
- US-A1- 2016 063 898
- No further relevant documents disclosed
- GALLAB ET AL.: "Development of a Spherical Model with a 3D Microchannel: Application to Glaucoma Surgery", MICROMACHINES, vol. 10, no. 5, 30 April 2019 (2019-04-30), pages 297, XP055807384, Retrieved from the Internet <URL:https://www.ncbi.nlm.nih.gov/pmc/articles/PMC6562714/pdf/micromachines-10-00297.pdf> [retrieved on 20201105]

## Description

### CROSS REFERENCE TO RELATED APPLICATIONS

This application claims the benefit of priority of U.S. Provisional Application No. 62/902,274, filed September 18, 2019.

### TECHNICAL FIELD

The present disclosure pertains generally, but not by way of limitation, synthetic eyes for surgical training. More particularly, the present disclosure relates to a realistic synthetic eye that can replicate the visual and tactile feedback of real surgery. Specifically the synthetic eye is designed for training/practice of inserting an implantable device for the treatment of glaucoma.

### BACKGROUND

According to a draft report by The National Eye Institute (NEI) at The United States National Institutes of Health (NIH), glaucoma is now the leading cause of irreversible blindness worldwide and the second leading cause of blindness, behind cataract, in the world. Thus, the NEI draft report concludes, "it is critical that significant emphasis and resources continue to be devoted to determining the pathophysiology and management of this disease." Glaucoma researchers have found a strong correlation between high intraocular pressure and glaucoma. For this reason, eye care professionals routinely screen patients for glaucoma by measuring intraocular pressure using a device known as a tonometer. Many modern tonometers make this measurement by blowing a sudden puff of air against the outer surface of the eye.

The eye can be conceptualized as a ball filled with fluid. There are two types of fluid inside the eye. The cavity behind the lens is filled with a viscous fluid known as vitreous humor. The cavities in front of the lens are filled with a fluid know as aqueous humor. Whenever a person views an object, he or she is viewing that object through both the vitreous humor and the aqueous humor.

Whenever a person views an object, he or she is also viewing that object through the cornea and the lens of the eye. In order to be transparent, the cornea and the lens can include no blood vessels. Accordingly, no blood flows through the cornea and the lens to provide nutrition to these tissues and to remove wastes from these tissues. Instead, these functions are performed by the aqueous humor. A continuous flow of aqueous humor through the eye provides nutrition to portions of the eye (e.g., the cornea and the lens) that have no blood vessels. This flow of aqueous humor also removes waste from these tissues.

Aqueous humor is produced by an organ known as the ciliary body. The ciliary body includes epithelial cells that continuously secrete aqueous humor. In a healthy eye, a stream of aqueous humor flows out of the anterior chamber of the eye through the trabecular meshwork and into Schlemm's canal as new aqueous humor is secreted by the epithelial cells of the ciliary body. This excess aqueous humor enters the venous blood stream from Schlemm's canal and is carried along with the venous blood leaving the eye.

When the natural drainage mechanisms of the eye stop functioning properly, the pressure inside the eye begins to rise. Researchers have theorized prolonged exposure to high intraocular pressure causes damage to the optic nerve that transmits sensory information from the eye to the brain. This damage to the optic nerve results in loss of peripheral vision. As glaucoma progresses, more and more of the visual field is lost until the patient is completely blind.

Ocular stents, scaffolds, or support structures can be implanted into Schlemm's canal for the purpose of lowering intraocular pressure by facilitating the flow of aqueous humor from the anterior chamber into Schlemm's canal. These ocular implants are typically delivered into Schlemm's canal by inserting a cannula of a delivery system into the anterior chamber of the eye, through the trabecular meshwork, and advancing the implant through the cannula into Schlemm' s canal. Upon implantation, the device provides a support structure, serving to dilate and restore the natural aqueous outflow pathway through Schlemm's canal, leading to a reduction of intraocular pressure.

Surgical training for delivery of ocular implants typically requires the use of human cadaver anterior segments. Prospective surgeons can practice delivery of ocular implants through the trabecular meshwork and into Schlemm's canal of eye bank eyes during the wet-lab portion of the training. However, human anterior segments are not easy to obtain, and are costly and difficult to transport and dispose of. Currently existing eye models do not properly replicate the feel and look of a human trabecular meshwork.
Reference is made to the documents US 2009/291423 A1 and Gallab et al.: "Development of a Spherical Model with a 3D Microchannel: Application to Glaucoma Surgery", Micromachines vol. 10, no. 5 30 April 2019 (2019-04-30), page 297 which have been cited as exemplary of the background state of the art.

### SUMMARY

The scope of the invention is in accordance with the appended claims.

A synthetic eye model is provided, comprising a rigid or semi-rigid eye shell, a synthetic iris base positioned within the eye shell, an eye core positioned within the eye shell and encircling the synthetic iris base, the eye core including a channel integral to the eye core configured to replicate Schlemm's canal of a human eye, and a synthetic trabecular meshwork tissue coupled to the eye shell and extending across the channel.

In some embodiments, the synthetic eye further comprises a synthetic cornea disposed over the eye shell.

According to the invention, the eye core further includes a protrusion adjacent to the channel and being configured to replicate a scleral spur of a human eye. According to the invention, the synthetic trabecular meshwork tissue extends across the protrusion and the channel.

In one embodiment, the synthetic trabecular meshwork comprises a hydrogel-based synthetic tissue.

In other embodiments, the rigid or semi-rigid eye shell includes a corneal portion and a scleral portion.

In some examples, the corneal portion integral to the scleral portion.

In one implementation, the eye core comprises a plurality of eye core pieces.

In another example, the synthetic eye includes one or more internal supports configured to attach to the synthetic trabecular meshwork tissue to prevent the synthetic trabecular meshwork tissue from collapsing into the channel.

In some embodiments, the synthetic eye further comprises a chamber positioned between the eye shell and the synthetic trabecular meshwork tissue.

A method performing surgical training is provided, comprising the steps of inserting an ocular implant into a synthetic eye, the ocular implant comprising an inlet portion at a proximal end and a Schlemm's canal portion disposed distal to the inlet portion, advancing the Schlemm' s canal portion of the implant through a synthetic trabecular meshwork tissue, advancing the Schlemm's canal portion of the implant into a channel of the synthetic eye, so that the Schlemm's canal portion tracks the channel as it is advanced, and disposing the inlet portion of the implant in a chamber of the synthetic eye adjacent to the synthetic trabecular meshwork tissue.

A method of deploying an ocular implant into a synthetic eye is provided, comprising piercing a synthetic trabecular meshwork with a distal tip of a delivery tool from within a chamber of the synthetic eye, inserting the distal tip of the delivery tool into a channel of the synthetic eye, and advancing an ocular implant from the delivery tool to place a body portion of the ocular implant in the channel and an inlet portion of the ocular implant in the chamber.

### BRIEF DESCRIPTION OF THE DRAWINGS

The disclosure may be more completely understood in consideration of the following detailed description of various embodiments in connection with the accompanying drawings, in which:
FIG. 1 illustrates one embodiment of synthetic eye model.
FIG. 2 is a cross-sectional view of an eye core and synthetic trabecular meshwork tissue of the synthetic eye model of FIG. 1.
FIGS. 3A-3B are view of a synthetic eye model.
FIG. 4A illustrates an embodiment of the synthetic eye model with a synthetic cornea.
FIG. 4B illustrates an embodiment of the synthetic eye model without a synthetic cornea, for an "open sky" approach.
FIG. 5 illustrates one embodiment of a synthetic eye model packaged in a representation of a human face.
FIGS. 6 and 7 illustrate alternative embodiments of an eye core of a synthetic eye model.
FIGS. 8-9 illustrate methods of performing surgical training on a synthetic eye.

While the disclosure is amenable to various modifications and alternative forms, specifics thereof have been shown by way of example in the drawings and will be described in detail. It should be understood, however, that the intention is not to limit the invention to the particular embodiments described.

### DETAILED DESCRIPTION

The following description should be read with reference to the drawings, which are not necessarily to scale, wherein like reference numerals indicate like elements throughout the several views. The detailed description and drawings are intended to illustrate but not limit the claimed invention. Those skilled in the art will recognize that the various elements described and/or shown may be arranged in various combinations and configurations without departing from the scope of the disclosure. The detailed description and drawings illustrate example embodiments of the claimed invention.

The following detailed description should be read with reference to the drawings, in which similar elements in different drawings are identified with the same reference numbers.
The drawings, which are not necessarily to scale, depict illustrative embodiments and are not intended to limit the scope of the disclosure.

The challenge with designing a synthetic model of the eye is producing a synthetic trabecular meshwork that properly replicates the feel and look of a human trabecular meshwork. The human trabecular meshwork is a thin delicate structure with membrane like properties. It is fragile and thin, but also stretchy and resilient. The appearance of the trabecular meshwork can vary with pigment amount and location within the angle.

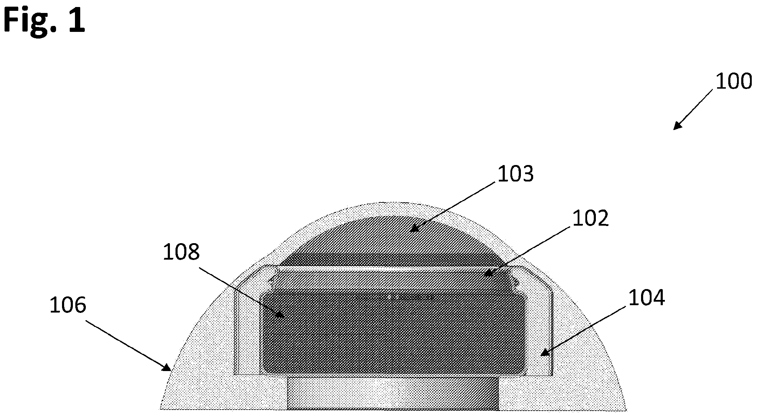

According to the present disclosure, a synthetic eye model includes several design elements. Referring to FIG. 1, a synthetic eye model 100 can include a synthetic trabecular meshwork tissue 102. Next, the synthetic eye model of the present disclosure includes an eye core 104 that includes many anatomical features, such as a scleral spur landmark, the back wall of Schlemm's canal, and an attachment for the synthetic trabecular meshwork. The synthetic eye model can further include an eye shell 106 configured to provide support for the eye core and provide cannula access through either a synthetic cornea or through an open design. In some embodiments, the eye shell 106 can provide an volume or chamber 103 designed and configured to represent the anterior chamber of the eye. This chamber 103 can be adjacent to the eye core 104, including the synthetic trabecular meshwork. The volume or chamber 103 can be empty (e.g., filled with air) or alternatively can be filled or injected with a fluid or substance with properties designed and configured to mimic aqueous humor in a real eye. Finally, the synthetic eye model can include an iris base 108 configured to provide a stable base and support for the eye core and to provide a visual reference for the scleral spur landmark. The synthetic eye model provided herein, when compared to a real human eye, includes proportional dimensions, realistic appearance, and realistic interactions between the cannula and ocular implant.

The synthetic trabecular meshwork tissue 102 can comprise a synthetic tissue to replicate the feel and appearance of real trabecular meshwork tissue. When the synthetic eye model 100 is fully assembled, the synthetic trabecular meshwork tissue 102 attaches to, connects to, or is stretched or wrapped around the eye core 104. In a real human eye, the trabecular meshwork is an area of tissue in the eye located around the base of the cornea, responsible for draining aqueous humor from the anterior chamber of the eye. A real trabecular meshwork tissue is "spongy" and lined by trabeculocytes which allows fluid drainage. In some examples, the synthetic trabecular meshwork tissue 102 comprises a hydrogel-based synthetic tissue material, such as SynDaver synthetic tissues, called SynTissue^{®}.

In some embodiments, the entire synthetic model of the eye is disposable and is intended for only a limited amount of training sessions. However, in other embodiments, many components of the synthetic eye model 100 are intended to be reused for future training sessions, with components such as the synthetic trabecular meshwork tissue and/or eye core being replaceable within a reusable eye shell. For example, the eye core may be modular within the eye shell, and can be removed when the synthetic trabecular meshwork tissue is in need of replacement. A new synthetic trabecular meshwork tissue may be applied or affixed to the eye core, and re-inserted into the eye shell for additional training sessions.

The eye core 104 includes features that, in combination with the synthetic trabecular meshwork tissue 104, combines to replicate look and feel of anatomical locations in a real eye such as Schlemm's canal. As described above, in a real eye the trabecular meshwork provides for drainage of aqueous humor from the anterior chamber into Schlemm's canal. Thus, in the synthetic eye model 100, the eye core 104 includes a cutout, groove, ridge, or channel that replicates the look and feel of a real Schlemm's canal. The eye core 104, as described above, can be configured to attach or connect to the synthetic trabecular meshwork tissue. In some embodiments, the eye core 104 can include anatomical features to improve the realism of the synthetic eye model. In one example, the eye core 104 can comprise a soft material such as silicon. However, it should be understood that other materials, including materials that mimic the softness, feel, and durability of real human tissues can be used.

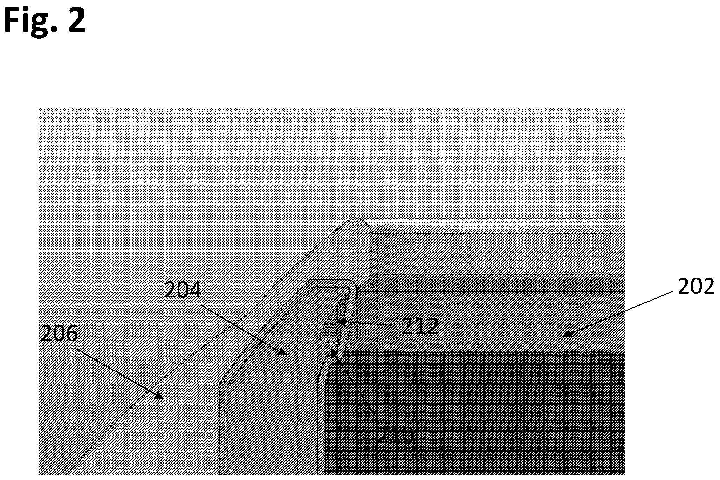

FIG. 2 is a close, cross-sectional view of the synthetic eye model of FIG. 1, including the synthetic trabecular meshwork tissue 202, the eye core 204, and the eye shell 206. As shown in the cross-sectional view, the eye core 204 reveals a protrusion 210 representing a real scleral spur and a channel 212 in the eye core 204 representing Schlemm's canal of a real eye. The synthetic trabecular meshwork 202 can be stretched and/or wrapped around the eye core 204. In another embodiment, the synthetic trabecular meshwork is stretched, wrapped, or positioned only across the protrusion 210 and channel 212. Although FIGS. 1 and 2 show cross-sectional views of the eye core, it should be understood that the eye core and/or trabecular meshwork tissue can be designed and configured to encircle the full 360 degrees around the iris base of the synthetic eye model. As shown in FIG. 2, the synthetic trabecular meshwork tissue 202 is coupled to and surrounds the eye core 204, including spanning from the protrusion 210 (representing the scleral spur) across the channel 212 representing Schlemm's canal. The synthetic trabecular meshwork tissue 202 can be stretched across the channel and the protrusion so as to form an open space within the channel 212 that replicates the natural anatomical structure of Schlemm's canal and the trabecular meshwork in a real human eye.

The eye core 104 (and 204 in FIG. 2) can be modular in design and is intended to be fitted or inserted into the eye shell 106. In some embodiments, the synthetic trabecular meshwork tissue 102, the eye core 104, and the iris base 106 can all be removable from the eye shell 108, either individually or interconnected. In one embodiment, the eye core 104/204 can be a single piece configured to encircle the full 360 degrees around the iris base of the synthetic eye model. However, in other embodiments, the eye core can comprise multiple pieces that join, connect, or abut one another to form the full 360 degree component as shown.

Referring back to FIG. 1, the eye shell 106 can be a rigid, semi-rigid material, or soft material configured and designed to hold and support the other components of the synthetic eye model. The eye shell 106 can be shaped and configured to mimic the look and/or feel of the cornea and/or sclera of a real human eye. In one embodiment, the eye shell 106 can be a single piece, but in other embodiments a synthetic cornea portion can be attached or connected to a synthetic sclera portion. In one example, the eye shell 106 can comprise a soft material such as silicon. However, it should be understood that other materials, including materials that mimic the softness, feel, and durability of real human tissues can be used. As described above, in some embodiments the entire synthetic eye model can be disposable and/or single or limited use. However, in other embodiments, components such as the eye shell can be re-used, and disposable elements such as the synthetic trabecular meshwork tissue can be replaced and inserted into the eye shell as needed.

The iris base 108 is configured to provide a stable base and support for the eye core and also serve as a visual reference for the scleral spur of the eye core 104. The iris base can be manufactured from a synthetic material chosen to imitate the look and/or feel of a real human iris. In one example, the iris base 108 can comprise a soft material such as silicon. The iris base 108 can be shaped and sized to hold or be connected or attached to the eye core 104 and/or the eye shell 106. During surgical training, the iris base can be used as a landmark to help the trainee/surgeon identify the location of the scleral spur.

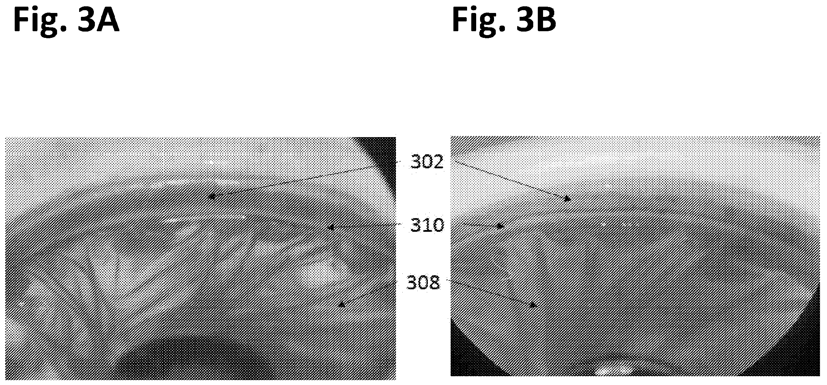

FIGS. 3A-3B are additional views of the synthetic eye model, showing the distinction between the synthetic trabecular meshwork 302, the scleral spur 310 of the eye core, and the iris base 306, as described above. It can be seen from FIGS. 3A-3B that the iris base 308 serves the important function of imitating the look of the human iris, but also providing the important visual contrast between the trabecular meshwork so as to identify the scleral spur, which is necessary for properly placing a delivery cannula within Schlemm's canal. The only difference between the two eye models in FIG. 3A and FIG. 3B are the techniques used to dye the canal/trabecular meshwork tissues, which will be described below.

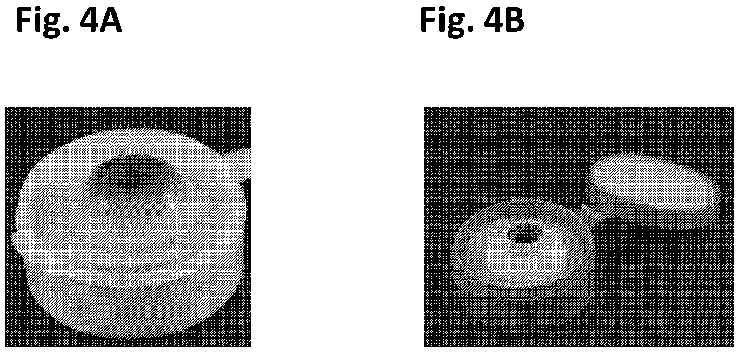

FIG. 4A illustrates a first configuration of a synthetic eye model which, in addition to the features described above, further includes a synthetic cornea that requires the use of an incision and a gonio lens to replicate the access and visualization method used in real surgery. In contrast, the a second configuration is illustrated in FIG. 4B, which represents an "open sky" approach without a synthetic cornea, allowing surgeons to directly visualize the trabecular meshwork and practice the cannula entry into Schlemm's canal without the need for the additional surgical steps of penetrating the cornea. As described above, in the first embodiment shown in FIG. 4A, the eye shell of the synthetic eye model can include the synthetic cornea, in either a one piece or two piece configuration. However, in FIG. 4B, the eye shell can include only a portion representing the sclera of the eye, with the cornea portion "missing" or removed for purposes of training.

As shown in both FIGS. 4A and 4B, the synthetic eye model can be packaged in a portable case for ease of shipping or transport. The case can include, for example, a hinged lid to allow ease of access to the synthetic eye. The design refinements needed for the synthetic eye model included molding modifications for reduction in flash, improved tissue quality control, and packaging design to maintain moisture in the tissue. Each synthetic eye model can be individually packaged in a sealed plastic container to retain moisture, keeping the synthetic trabecular meshwork tissue hydrated and realistically simulating the feel of a human trabecular meshwork.

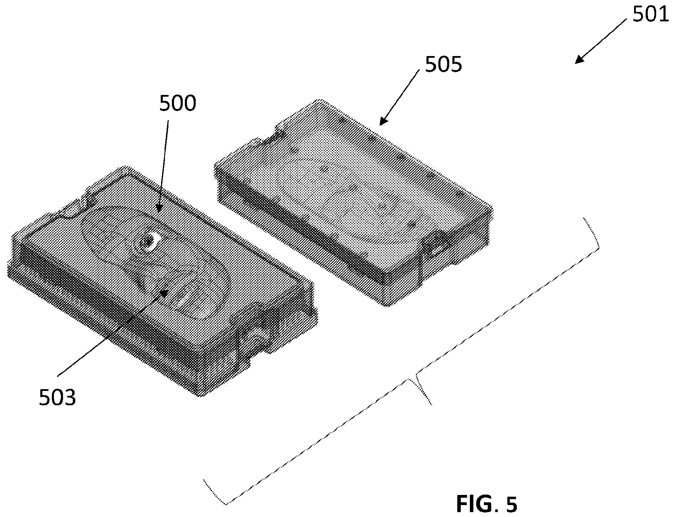

Referring to FIG. 5, a surgical training kit 501 can include the synthetic eye model 500 described herein inserted into a contoured tray 503 that represents a human face. The tray can fit into an instrument case 505 that serves as a convenient carrying case for various instruments, devices, and materials needed to complete the training. In one embodiment, the instrument case 505 can include a top half and a bottom half that close and seal together to protect the synthetic eye model 500 and the contoured tray 503. In use, the instrument case can be opened, exposing the synthetic eye model and contoured tray.

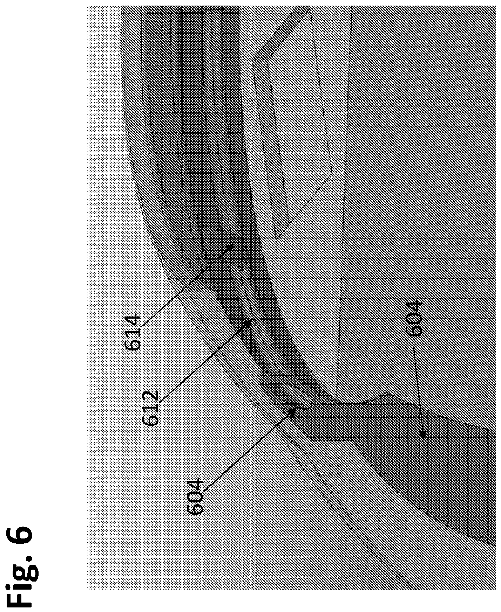

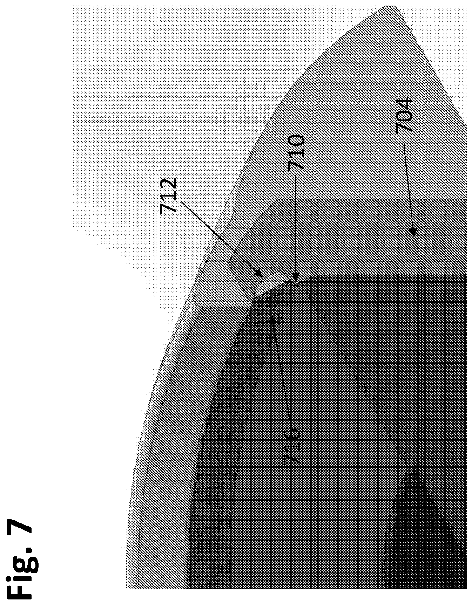

Alternative embodiments of the eye core and synthetic trabecular meshwork tissue are shown in FIGS. 6 and 7. Referring to FIG. 6, eye core 604 can include internal supports 614 configured to attach to a synthetic or real human trabecular meshwork tissue. The internal supports 614 can span or extend across the channel 612 of the eye core 604 to prevent the synthetic trabecular meshwork tissue from collapsing into the channel. Any number of internal supports can be used, depending on the material used for the eye core and the material used for the synthetic trabecular meshwork. In this embodiment, the synthetic or human trabecular meshwork tissue can be removed and/or replaced onto the eye core 604 as needed. It should be noted that in this embodiment, the eye core 604 includes a representation of Schlemm's canal, but the scleral spur landmark is not as pronounced as the embodiment of FIG. 2. It should be understood that this embodiment can also include the scleral spur landmark or protrusion as described above in FIG. 2.

Referring to FIG. 7, another embodiment of an eye core 704 includes the scleral spur landmark as represented by protrusion 710, and the channel 712 configured and shaped to represent Schlemm's canal. However, in this embodiment, a two-part gel adhesive 716 can be injected, placed, or inserted into the channel 712 to represent both the trabecular meshwork and Schlemm's canal. In this embodiment, a first gel adhesive is inserted into the channel with properties designed and configured to mimic the feel of Schlemm's canal, and then a second gel adhesive is applied on top of the first gel adhesive with properties designed and configured to mimic the feel of the trabecular meshwork. Contrary to the prior embodiments described above in which a synthetic trabecular meshwork is stretched or wrapped around the eye core, in this embodiment the synthetic material is injected into or placed within the channel of the eye core.

In some examples, the canal or channel portion within the eye core of the synthetic eye representing Schlemm's canal can be dyed to highlight the synthetic trabecular meshwork tissue as a further visual aid for the surgical training. In one embodiment, the canal is dyed with a raw silicone dye that is uncured and injected into the canal after assembly of the synthetic eye model. The uncured silicone dye does not dissipate over time since the hydrogel-based synthetic tissue of the trabecular meshwork only absorbs water, and the silicone of the eye core is hydrophobic. With this dye and method, the silicone dye stays in the canal and the area of tissue over the scleral spur landmark in the eye core remains clear. The synthetic eye model on the left of FIG. 3 illustrates a canal dyed with this technique.

In another embodiment a different dye mixture of larger dye particles is suspended in a gel like mixture of propylene glycol, glycerine, and iron oxide. The mixture is unique because the iron oxide particles are large and get stuck in the hydrogel much similar in appearance to a real human trabecular meshwork. Instead of being a solid brown line, the trabecular meshwork looks textured and speckled, making the synthetic tissue look remarkably realistic. Over time the iron oxide particles appear to maintain their position and concentration in the synthetic tissue. The synthetic eye model on the right of FIG. 3 illustrates a canal dyed with this technique.

As described above, the synthetic eye model can be used for surgical training to mimic the feel of inserting an ocular implant into Schlemm's canal. In some embodiments, the ocular implant can be sized and shaped to fit within Schlemm's canal (or within the channel of the synthetic eye model). The ocular implant can include a curved body that can be generally tubular in shape and can include one or more openings along its length. In some embodiments, the ocular implant is designed and configured to reside fully within Schlemm's canal (or the synthetic Schlemm's canal). In other embodiments, the ocular implant can include an inlet portion that is designed and configured to extend from Schlemm's canal into the anterior chamber.

The ocular implants used for surgical training with the synthetic eye model typically will require or incorporate a delivery system. The delivery system can be configured to advance the ocular implant into the eye of a patient, or in this case, into the synthetic eye model. Typically, these ocular implant delivery systems will include a cannula configured to house the ocular implant and an advancement system or mechanism configured to push or advance the ocular implant out of the cannula or into the eye (or synthetic eye). In some embodiments, the delivery system can include features that improve the ease of use, including, for example, cutting distal portions on the cannula for insertion into the eye and/or rotation features or curvature designed and configured to facilitate proper orientation with between the cannula and the target tissue (e.g., Schlemm's canal).

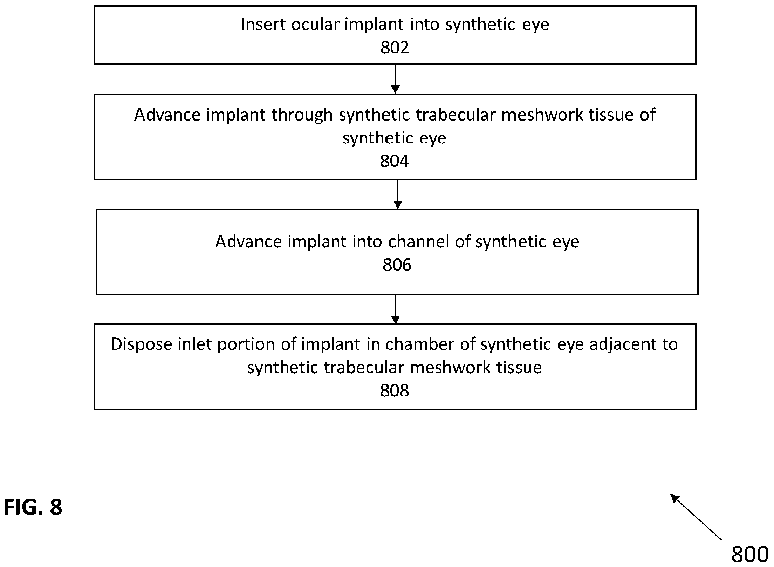

Methods of use are also provided herein. FIG. 8 is a flowchart 800 that describes a method performing surgical training. At step 802, the method can include inserting an ocular implant into a synthetic eye. This method step can include, for example, inserting the ocular implant through an eye shell of the synthetic eye. The eye shell can include a corneal portion and/or a scleral portion. In one embodiment, the ocular implant is inserted through the corneal portion into a chamber of the synthetic eye. As described above, any type of ocular implant can be used with the synthetic eye, but in some embodiments the synthetic eye is suited for ocular implants that are designed and configured to be inserted into Schlemm's canal of a human eye. In one example, the ocular implant comprising an inlet portion at a proximal end and a Schlemm's canal portion disposed distal to the inlet portion.

Next, at step 804, the method can include advancing the Schlemm's canal portion of the implant through a synthetic trabecular meshwork tissue. At step 806, the method can include advancing the Schlemm's canal portion of the implant into a channel of the synthetic eye, so that the Schlemm's canal portion tracks the channel as it is advanced. As described above, the synthetic trabecular meshwork tissue can be stretched or extended across a channel of the eye core. In some examples, the synthetic meshwork tissue stretches across a protrusion representing the scleral spur and across the channel.

Finally, at step 806, the method can include disposing the inlet portion of the implant in a chamber of the synthetic eye adjacent to the synthetic trabecular meshwork tissue. As described above, the chamber of the synthetic eye can be designed and configured to represent an anterior chamber of a human eye. In this method, the inlet or proximal portion of the ocular implant is implanted so as to remain within the chamber of the synthetic eye, while the Schlemm's canal or distal portion of the implant is implanted to remain with the channel of the synthetic eye.

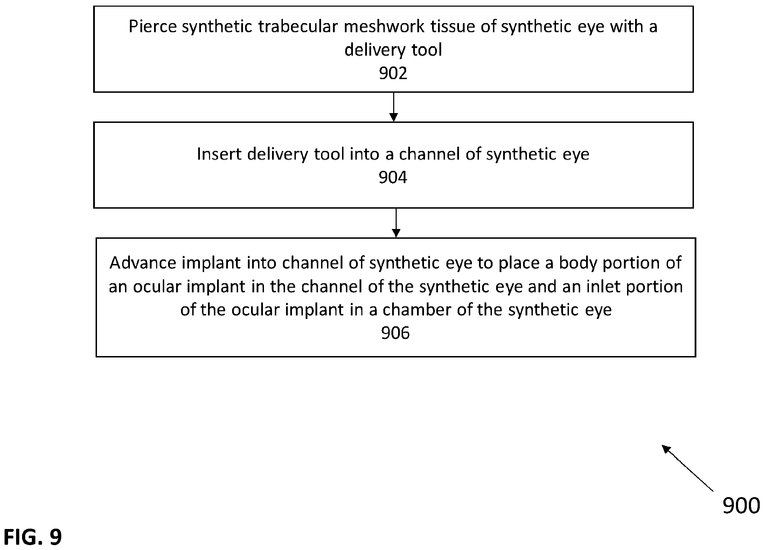

FIG. 9 is a flowchart 900 that describes a method performing surgical training. method of deploying an ocular implant into a synthetic eye. Referring to step 902, the method can include piercing a synthetic trabecular meshwork with a distal tip of a delivery tool from within a chamber of the synthetic eye. As described above, the delivery tool can include a cannula. In some embodiments, the cannula can include a distal tip designed and configured to pierce tissue. The tip can be sharp or a cutting tip, for example.

At step 904, the method can further include inserting the distal tip of the delivery tool into a channel of the synthetic eye. As described above, the channel of the eye core can be designed and configured to mimic the characteristics and dimensions of a real Schlemm's canal.

Finally, at step 902, the method can include advancing an ocular implant from the delivery tool to place a body portion of the ocular implant in the channel and an inlet portion of the ocular implant in the chamber. As described above, in some embodiments the ocular implant can include an inlet portion designed and configured to remain within the anterior chamber of the eye when the distal or body portion of the implant is inserted into Schlemm's canal, to facilitate the removal of aqueous humor from the anterior chamber.

Definitions of certain terms are provided below and shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same or substantially the same function or result). In many instances, the terms "about" may include numbers that are rounded to the nearest significant figure. Other uses of the term "about" (i.e., in a context other than numeric values) may be assumed to have their ordinary and customary definition(s), as understood from and consistent with the context of the specification, unless otherwise specified.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a," "an," and "the" include or otherwise refer to singular as well as plural referents, unless the content clearly dictates otherwise. As used in this specification and the appended claims, the term "or" is generally employed to include "and/or," unless the content clearly dictates otherwise.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to affect such feature, structure, or characteristic in connection with other embodiments, whether or not explicitly described, unless clearly stated to the contrary. That is, the various individual elements described below, even if not explicitly shown in a particular combination, are nevertheless contemplated as being combinable or able to be arranged with each other to form other additional embodiments or to complement and/or enrich the described embodiment(s), as would be understood by one of ordinary skill in the art.

It is to be understood that even though numerous characteristics of various embodiments have been set forth in the foregoing description, together with details of the structure and function of various embodiments, this detailed description is illustrative only, and the invention is defined in the appended claims.

## Claims

1. A synthetic eye model (100), comprising:
a rigid or semi-rigid eye shell (106);
a synthetic iris base (108) positioned within the eye shell (106);
an eye core (104, 604) positioned within the eye shell and encircling the synthetic iris base, the eye core including a channel (212) integral to the eye core (204) configured to replicate Schlemm' s canal of a human eye;
the synthetic eye model being **characterized in that** it further comprises:
a synthetic trabecular meshwork tissue (202) coupled to the eye shell (106) and extending across the channel (212); and
wherein the eye core (204) further including a protrusion (210) adjacent to the channel (212) and being configured to replicate a scleral spur of a human eye.

2. The synthetic eye model of claim 1, further comprising a synthetic cornea disposed over the eye shell (106).

3. The synthetic eye model of claim 1, wherein the synthetic trabecular meshwork tissue (202) extends across the protrusion and the channel.

4. The synthetic eye model of claim 1, wherein the synthetic trabecular meshwork tissue (202) comprises a hydrogel-based synthetic tissue.

5. The synthetic eye model of claim 1, wherein the rigid or semi-rigid eye shell includes a corneal portion and a scleral portion.

6. The synthetic eye model of claim 5, wherein the corneal portion is integral to the scleral portion.

7. The synthetic eye model of claim 1, wherein the eye core (104) comprises a plurality of eye core pieces.

8. The synthetic eye model of claim 1, further comprising one or more internal supports (614) configured to attach to the synthetic trabecular meshwork tissue (202) to prevent the synthetic trabecular meshwork tissue from collapsing into the channel (212).

9. The synthetic eye model of claim 1, further comprising a chamber (103) positioned between the eye shell (106) and the synthetic trabecular meshwork tissue (202).

10. A method of performing surgical training in a synthetic eye model as claimed in any of claims 1-9, comprising the steps of:
inserting (802) an ocular implant into a synthetic eye, the ocular implant comprising an inlet portion at a proximal end and a Schlemm's canal portion disposed distal to the inlet portion;
advancing (804) the Schlemm's canal portion of the implant through a synthetic trabecular meshwork tissue (202);
advancing (806) the Schlemm's canal portion of the implant into a channel of the synthetic eye, so that the Schlemm's canal portion tracks the channel as it is advanced; and
disposing the inlet portion of the implant in a chamber of the synthetic eye adjacent to the synthetic trabecular meshwork tissue.

11. A method of deploying an ocular implant into a synthetic eye, comprising:
piercing a synthetic trabecular meshwork (202) with a distal tip of a delivery tool from within a chamber of the synthetic eye;
inserting the distal tip of the delivery tool into a channel (212) of the synthetic eye (100); and advancing an ocular implant from the delivery tool to place a body portion of the ocular implant in the channel and an inlet portion of the ocular implant in the chamber.

## Patentansprüche

1. Synthetisches Augenmodell (100), umfassend:
eine starre oder halbstarre Augenschale (106);
eine synthetische Irisbasis (108), die innerhalb der Augenschale (106) positioniert ist;
einen Augenkern (104, 604), der innerhalb der Augenschale positioniert ist und die synthetische Irisbasis umgibt, wobei der Augenkern einen Kanal (212) umfasst, der aus einem Stück mit dem Augenkern (204) und dazu ausgelegt ist, den Schlemm-Kanal eines menschlichen Auges nachzubilden; wobei das synthetische Augenmodell **dadurch gekennzeichnet ist, dass** es ferner Folgendes umfasst:
ein synthetisches Trabekelwerkgewebe (202), das mit der Augenschale (106) gekoppelt ist und sich über den Kanal (212) erstreckt; und
wobei der Augenkern (204) ferner einen Vorsprung (210) benachbart zum Kanal (212) umfasst und dazu ausgelegt ist, einen Skleralsporn eines menschlichen Auges nachzubilden.

2. Synthetisches Augenmodell nach Anspruch 1, ferner umfassend eine synthetische Hornhaut, die über der Augenschale (106) angeordnet ist.

3. Synthetisches Augenmodell nach Anspruch 1, wobei sich das synthetische Trabekelwerkgewebe (202) über den Vorsprung und den Kanal erstreckt.

4. Synthetisches Augenmodell nach Anspruch 1, wobei das synthetische Trabekelwerkgewebe (202) ein synthetisches Gewebe auf Hydrogelbasis umfasst.

5. Synthetisches Augenmodell nach Anspruch 1, wobei die starre oder halbstarre Augenschale einen Hornhautabschnitt und einen Skleralabschnitt umfasst.

6. Synthetisches Augenmodell nach Anspruch 5, wobei der Hornhautabschnitt aus einem Stück mit dem Skleralabschnitt ist.

7. Synthetisches Augenmodell nach Anspruch 1, wobei der Augenkern (104) eine Mehrzahl von Augenkernstücken umfasst.

8. Synthetisches Augenmodell nach Anspruch 1, ferner umfassend eine oder mehrere interne Stützen (614), die dazu ausgelegt sind, am synthetischen Trabekelwerkgewebe (202) befestigt zu werden, um zu verhindern, dass das synthetische Trabekelwerkgewebe in den Kanal (212) zusammenfällt.

9. Synthetisches Augenmodell nach Anspruch 1, ferner umfassend eine Kammer (103), die zwischen der Augenschale (106) und dem synthetischen Trabekelwerkgewebe (202) positioniert ist.

10. Verfahren zum Durchführen eines chirurgischen Trainings in einem synthetischen Augenmodell nach einem der Ansprüche 1 bis 9, umfassend die folgenden Schritte:
Einführen (802) eines Augenimplantats in ein synthetisches Auge, wobei das Augenimplantat einen Einlassabschnitt an einem proximalen Ende und einen Schlemm-Kanal-Abschnitt umfasst, der distal zum Einlassabschnitt angeordnet ist;
Vorschieben (804) des Schlemm-Kanal-Abschnitts des Implantats durch ein synthetisches Trabekelwerkgewebe (202) ;
Vorschieben (806) des Schlemm-Kanal-Abschnitts des Implantats in einen Kanal des synthetischen Auges, so dass der Schlemm-Kanal-Abschnitt dem Kanal folgt, während er vorgeschoben wird; und
Anordnen des Einlassabschnitts des Implantats in einer Kammer des synthetischen Auges benachbart zum synthetischen Trabekelwerkgewebe.

11. Verfahren zum Einsetzen eines Augenimplantats in ein synthetisches Auge, umfassend:
Durchstechen eines synthetischen Trabekelwerks (202) mit einer distalen Spitze eines Zuführwerkzeugs von innerhalb einer Kammer des synthetischen Auges;
Einführen der distalen Spitze des Zuführwerkzeugs in einen Kanal (212) des synthetischen Auges (100); und
Vorschieben eines Augenimplantats von dem Zuführwerkzeug, um einen Körperabschnitt des Augenimplantats im Kanal und einen Einlassabschnitt des Augenimplantats in der Kammer zu platzieren.

## Revendications

1. Modèle d'œil synthétique (100), comprenant :
une enveloppe d'œil rigide ou semi-rigide (106) ;
une base d'iris synthétique (108) positionnée à l'intérieur de l'enveloppe d'œil (106) ;
un coeur d'œil (104, 604) positionné à l'intérieur de l'enveloppe d'œil et entourant la base d'iris synthétique, le coeur d'œil comprenant un canal (212) intégré au coeur d'œil (204) configuré pour reproduire le canal de Schlemm d'un œil humain ; le modèle d'œil synthétique étant **caractérisé en ce qu'**il comprend en outre :
un tissu de réseau trabéculaire synthétique (202) couplé à l'enveloppe d'œil (106) et s'étendant à travers le canal (212) ; et
le coeur d'œil (204) comprenant en outre une protubérance (210) adjacente au canal (212) et
configurée pour reproduire un éperon scléral d'un œil humain.

2. Modèle d'œil synthétique selon la revendication 1, comprenant en outre une cornée synthétique disposée sur l'enveloppe d'œil (106).

3. Modèle d'œil synthétique selon la revendication 1, le tissu de réseau trabéculaire synthétique (202) s'étendant à travers la protubérance et le canal.

4. Modèle d'œil synthétique selon la revendication 1, le tissu de réseau trabéculaire synthétique (202) comprenant un tissu synthétique à base d'hydrogel.

5. Modèle d'œil synthétique selon la revendication 1, l'enveloppe d'œil rigide ou semi-rigide de l'œil comprenant une partie cornéenne et une partie sclérale.

6. Modèle d'œil synthétique selon la revendication 5, la partie cornéenne étant intégrée à la partie sclérale.

7. Modèle d'œil synthétique selon la revendication 1, le coeur d'œil (104) comprenant une pluralité de parties du coeur d'œil.

8. Modèle d'œil synthétique selon la revendication 1, comprenant en outre un ou plusieurs supports internes (614) configurés pour s'attacher au tissu de réseau trabéculaire synthétique (202) afin d'empêcher le tissu de réseau trabéculaire synthétique de se replier dans le canal (212).

9. Modèle d'œil synthétique selon la revendication 1, comprenant en outre une chambre (103) positionnée entre l'enveloppe d'œil (106) et le tissu de réseau trabéculaire synthétique (202).

10. Procédé d'entraînement chirurgical dans un modèle d'œil synthétique selon l'une quelconque des revendications 1 à 9, comprenant les étapes suivantes :
l'insertion (802) d'un implant oculaire dans un œil synthétique, l'implant oculaire comprenant une partie d'entrée à une extrémité proximale et une partie du canal de Schlemm disposée distalement par rapport à la partie d'entrée ;
l'avancement (804) de la partie du canal de Schlemm de l'implant à travers un tissu de réseau trabéculaire synthétique (202) ;
l'avancement (806) de la partie du canal de Schlemm de l'implant dans un canal de l'œil synthétique, de sorte que la partie du canal de Schlemm suive le canal au fur et à mesure de l'avancement ; et
la disposition de la partie d'entrée de l'implant dans une chambre de l'œil synthétique adjacente au tissu de réseau trabéculaire synthétique.

11. Procédé de déploiement d'un implant oculaire dans un œil synthétique, comprenant :
le percement d'un réseau trabéculaire synthétique (202) avec l'extrémité distale d'un outil d'insertion à l'intérieur d'une chambre de l'œil synthétique ;
l'insertion de l'extrémité distale de l'outil d'insertion dans un canal (212) de l'œil synthétique (100) ; et
l'avancement d'un implant oculaire à partir de l'outil d'insertion pour placer une partie du corps de l'implant oculaire dans le canal et une partie d'entrée de l'implant oculaire dans la chambre.
